(19) Europäisches Patentamt
European Patent Office
Office européen des brevets

(11) Publication number: **0 180 045**
**A2**

(12) **EUROPEAN PATENT APPLICATION**

(21) Application number: **85112352.1**

(22) Date of filing: **30.09.85**

(51) Int. Cl.⁴: **C 12 P 19/56**, A 61 K 31/70, C 07 H 15/24 // (C12P19/56, C12R1:365)

(30) Priority: **28.09.84 US 655962**
**16.09.85 US 775218**

(43) Date of publication of application: **07.05.86**
**Bulletin 86/19**

(84) Designated Contracting States: **BE CH DE FR GB IT LI NL SE**

(71) Applicant: **ABBOTT LABORATORIES, 14th Street and Sheridan Road North St, North Chicago Illinois 60064 (US)**

(72) Inventor: **Theriault, Robert John, Sr., 6044 47th Avenue, Kenosha, WI 53140 (US)**
Inventor: **Rasmussen, Ronald Robert, 536 North Lewis, Waukegan, IL (US)**

(74) Representative: **Modiano, Guido et al, MODIANO, JOSIF, PISANTY & STAUB Modiano & Associati Via Meravigli, 16, I-20123 Milan (IT)**

(54) **Benzanthrin compounds.**

(57) New compounds, benzanthrins, are produced by a microorganism belonging to the genus Nocardia. The compounds are effective Gram-positive antibiotics and are cytotoxic.

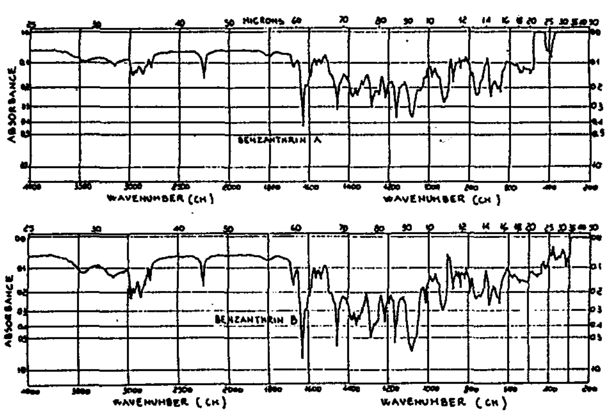


EP 0 180 045 A2

# BENZANTHRIN COMPOUNDS

## Technical Field

This invention relates to new antibiotic and cytotoxic agents and a process for making the same.

## Background Art and Disclosure of the Invention

Numerous antibiotics in the prior art, which exhibit activity against a broad spectrum of Gram-positive bacteria, have been produced from species of microorganisms which have been isolated from soil samples throughout the world. Such antibiotics continue to be in demand.

Further, cytotoxic compounds are in demand, particularly anti-cancer compounds.

The new compounds of the present invention have been named benzanthrin. They are believed to be of the following chemical structure:

(I)

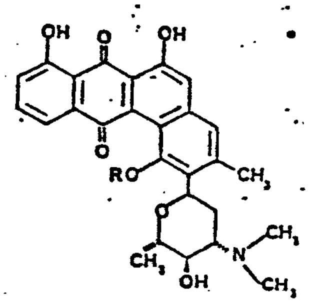

wherein R is

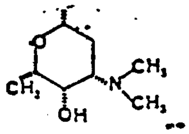

or

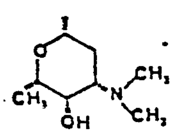

The benzanthrins exhibit antibacterial activity against various Gram-positive bacteria and cytotoxic activity.

The benzanthrins are produced by a microorganism belonging to the genus *Nocardia* in yields of about 3 mcg/ml. These benzanthrins are produced by growing the culture in a nutrient medium. Thereafter, once isolated from the culture medium by solvent extraction with a water immiscible organic solvent followed by chromatography, the compounds of the invention are in substantially pure form.

## Brief Description of the Drawings

FIGURE 1 illustrates the infrared absorption spectra of benzanthrins A and B in chloroform.

FIGURE 2 illustrates the hydrogen nuclear magnetic resonance spectra of benzanthrins A and B in methylene chloride-$d_2$.

FIGURE 3 illustrates the carbon-13 nuclear magnetic resonance spectra of benzanthrins A and B in methylene chloride-$d_2$.

FIGURE 4 illustrates the infrared absorption in chloroform, hydrogen nuclear magnetic resonance and carbon-13 nuclear magnetic resonance spectra for benzanthrin pseudoaglycone in methylene chloride-$d_2$.

## Best Mode for Carrying Out the Invention

The benzanthrins are produced by fermentation of a microorganism belong to the genus *Nocardia*. A particularly suitable microorganism is a phage resistant mutant of *Nocardia lurida* designated B-7. This strain has been deposited in the Agricultural Research Service Culture Collection at Northern Regional Research Center, 1815 North University Street, Peoria, Illinois 61604 and has been given the accession number NRRL 15820.

I. Morphology and Culture Characteristics:

Nocardia lurida B7 is a single clone isolated as a phage resistant variant of Nocardia lurida NRRL 2430. Nocardia lurida NRRL 2430 is described by Philip and Schenck in U.S. Patent No. 2,990,329 issued June 27, 1961, and by Pridham and Lyons, Developments in Industrial Microbiology, Vol. 10, p. 208 (1969). N. lurida grows with intertwined, branched mycelium which is non-septate initially but segments in 48 hours and is completely fragmented by 72 to 96 hours. The fragments are smooth-walled, rod-shaped, 0.8 to 1.5 microns in length with well-defined ends. The mycelium is approximately 0.5 micron in diameter. The aerial mycelium is white. The vegetative mycelium is a light golden orange color and is heavily crinkled on agar surfaces. N. lurida utilizes glucose, xylose, arabinose, fructose, galactose, D-mannitol and salicin. It is unable to grow when Rhamnose, sucrose, raffinose, starch or inositol are the sole carbon source. N. lurida contains the meso form of diaminopimelic acid. The major sugars in whole cell hydrolysates are arabinose and galactose. N. lurida is not acid-fast and does not contain mycolic acids.

II. Fermentation:

Cultivation of strain B-7 for the production of benzanthrins is carried out in liquid media. An assimilable carbon source such as glucose is used in combination with an organic nitrogen source such as peptone, lactalbumin, spray dried lard water, NZ amine, pepticase, soytone plus yeast extract or whole yeast and sodium chloride. Calcium carbonate is added to aid in controlling the pH of the fermentation. Other organic and inorganic ingredients may be added to stimulate production of the compounds of the invention.

A liquid, submerged, stirred culture process is preferred for the production of the compounds of the invention. Fermentations are carried out at a temperature range of 23°C to 28°C. The pH of the fermentation is preferably maintained between 6 and 9. Compounds of the invention are produced and accumulated between 2 and 12 days after inoculation of the fermentation.

## Example 1

## Benzanthrin A

Culture B-7 was maintained on medium A26a agar slants, the ingredients for which are shown in Table 1.

A4B seed flasks (Table 1) were inoculated from A26a agar slant cultures of B-7. Seed flasks were incubated on a Gump rotary shaker (250 rpm) at 24°C for 48 hours. At that time, 5% vegetative inoculum was transferred aseptically to fermentation flasks containing the following ingredients:

| | gm/liter |
|---|---|
| Glucose monohydrate* | 30 |
| LEXEIN F-152 peptone (made by Inolex Chemical Co., Chicago, Illinois) | 20 |
| Primary NF yeast (made by Yeast Products, Inc., Clinton New Jersey) | 5 |
| Sodium chloride (NaCl) | 5 |
| Calcium carbonate ($CaCO_3$) | 1 |

no pH adjustment
cold tap $H_2O$ QS to 1.0 liter

Vol/flask: 50 ml/500 ml Erlenmeyer flask closed with cotton plugs

Sterilization: 30 min, 121°C, 15-16 lb pressure

*Sterile glucose monohydrate was added post-sterilization.

Fermentation flasks were incubated on a Gump rotary shaker (250 rpm) at 24°C for 72 hours. At that time, the fermentation beer (50 ml) was adjusted to pH 6, saturated with sodium chloride and then extracted twice with 1/4 volume of n-butanol. The butanol extracts were combined, evaporated to dryness under vacuum and reconstituted in methanol. The methanol solution of the butanol extract residue was applied to a cellulose sheet made by Eastman Kodak Co., Rochester, New York, which was developed upflow in a solvent system consisting of 1% aqueous p-toluene- sulfonic acid saturated with methylisobutylketone. After development, the cellulose sheet was dried 24-48 hours and placed on a bioautograph plate seeded with Staphylococcus aureus ATCC 6538P. The bioautograph plate was incubated overnight at 32°C. Antibiotic activity was observed at $R_f$ 0.5 (relative mobility of sample/solvent front). Yellow/orange color on the cellulose sheet coincided with the antibiotic activity.

This same solution was also applied to a silica gel, thin-layer (Merck GF 254) plate which was developed in a solvent system consisting of n-butanol: n-propanol:acetic acid:water:pyridine (25:5:3:32:10). After development, the thin-layer plate was dried and examined with UV light (254 nm) revealing two intense UV absorbing products at $R_f$ 0.28 and 0.31. The two products were visibly colored yellow/orange.

## Example 2

## Benzanthrin A

A48 seed flasks (Table 1) were inoculated from A26a agar slant cultures of B-7. Seed flasks were incubated on a Gump rotary shaker (250 rpm) at 24°C for 48 hours. At that time, 5% vegetative inoculum was transferred aseptically to 300 fermentation flasks containing the following ingredients:

| | gm/liter |
|---|---|
| glucose monohydrate* | 30 |
| RED STAR yeast (made by Universal Foods, Milwaukee, Wisconsin) | 5 |
| LEXEIN F-152 peptone | 20 |
| NaCl | 5 |
| $CaCO_3$ | 1 |

no pH adjustment

cold tap $H_2O$ QS to 1.0 liter

Vol/flask: 50 ml/500 ml Erlenmeyer flask closed with cotton plugs

Sterilization: 30 min, 121°C, 15-16 lb pressure

*Sterile glucose monohydrate was added post-sterilization.

Fermentation flasks were incubated on a Gump rotary shaker (250 rpm) at 24°C for 72 hours at which time the fermentation beer in the flasks was harvested and pooled. A 50 ml aliquot of the fermentation beer was adjusted to pH 7, saturated with sodium chloride and extracted twice with 1/4 volume of n-butanol. The butanol extracts were combined and dried under vacuum and reconstituted in methanol. A second 50 ml aliquot of the fermentation beer was adjusted to pH 8.5. The beer was centrifuged, and the supernate was extracted three times with 1/2 volume of methylene chloride. The methylene chloride extracts were combined, dried under vacuum and reconstituted in methanol. The butanol and methylene chloride extracts were applied to silica gel, thin-layer (Analtech GF) plates which were developed in three solvent systems. After development, the plates were dried and examined with visible and short-wave UV light. The thin-layer plates were then placed on

bioautograph agar plates seeded with Staphylococcus aureus ATCC 6538P for 1 hour. The thin-layer plates were removed and the bioautograph plates were placed in a 32°C incubator overnight. The bioautograph plates were then examined for the presence of antibiotic activity. In three solvent systems as indicated in Table 2, an antibiotic active against Staphylococcus aureus was observed. This compound was yellow/orange in color and absorbed short-wave UV light.

## Example 3

## Benzanthrin A

A4B seed flasks (Table 1) were inoculated from A26a agar slant cultures of B-7. Seed flasks were incubated on a Gump rotary shaker (250 rpm) at 24°C for 48 hours. At that time, 5% vegetative inoculum was transferred aseptically to sterile 14 liter stainless steel fermentors containing the following medium and conditions:

| | gm/liter |
|---|---|
| glucose monohydrate* | 30 |
| RED STAR yeast | 5 |
| LEXEIN F-152 peptone | 20 |
| NaCl | 5 |
| $CaCO_3$ | 1 |

no pH adjustment

distilled $H_2O$ QS to 1.0 liter

Antifoam: .01% P-2000 polyethyleneglycol (Dow Chemical Co., Midland, Michigan)

Sterilization Time: 1 hour, 121°C, 15-16 lb pressure

Agitation: 200 RPM

Blade angle: 90°

Air Rate: 1 volume/volume/min

Incubation Temp: 24°C

*Sterile glucose monohydrate was added post sterilization.

Fermentors were incubated for 9 days. Fifty ml samples of fermentation beer were taken daily for both butanol and methylene chloride extractions.
The extracts were chromatographed on silica gel, thin-layer (Analtech GF) plates. As described in Example 2, a yellow/orange colored antibiotic active against *Staphylococcus aureus*, was observed at 90 hr, 114 hr and 138 hr in the fermentation.

## Example 4

## Benzanthrin A and B

Culture B-7 was maintained on medium A26a agar plates, the ingredients for which are shown in Table 1.

A4B seed flasks (Table 1) were inoculated from A26a agar slant cultures of B-7. Seed flasks were incubated on a Gump rotary shaker (250 rpm) at 24°C for 48 hours. At that time, 5% vegetative inoculum was transferred aseptically to fermentation flasks containing the following ingredients:

| | gm/liter |
|---|---|
| glucose monohydrate* | 30 |
| LEXEIN F-152 peptone (made by Inolex Chemical Co., Chicago, Illinois) | 20 |
| NaCl | 5 |
| $CaCO_3$ | 1 |
| no pH adjustment | |

distilled $H_2O$ q.s. to 1.0 liter

Vol/flask: 50 ml/500 ml Erlenmeyer flask closed with rayon plugs

Sterilization: 30 min, 121°C, 15-16 lb pressure

*Sterile glucose monohydrate was added post-sterilization.

Fermentation flasks were incubated on a Gump rotary shaker (250 rpm) at 24°C for 72 hours at which time the fermentation beer in the flasks was harvested. A 50 ml aliquot of the harvested fermentation beer was adjusted to pH 8.5 and then extracted three times with 1/2 volume of methylene chloride. The methylene chloride extracts were combined, dried under vacuum and reconstituted in methanol. The methylene chloride extracts (in methanol) were applied to silica gel (Analtech GF) thin-layer plates. A methanolic saturated sodium chloride solution was also applied to each extract on the silica gel thin-layer plates. The thin-layer plates were then developed in a solvent system consisting methylene chloride:methanol:water (70:30:1) for 45 minutes and then dried. Two orange colored products were observed at $R_f$ 0.15 and 0.23 under visible light which also absorbed short-wave UV light. ..e thin-layer plates were then placed on bioautograph plates seeded with Staphylacoccus aureus ATCC 6538P for 1 hour. The thin-layer plates were removed and the bioautograph plates were placed in a 32°C incubator overnight. The bioautograph plates were then examined for antibiotic activity. Two compounds which inhibited Staphylacoccus aureus were observed at $R_f$ 0.15 and 0.23 which coincided with the two orange colored UV absorbing products.

Table 1

A26A Slant Medium

| | gm/liter |
|---|---|
| glycerol | 10. |
| L-arginine | 2.5 |
| NaCl | 1. |
| $K_2HPO_4$ | 1. |
| $FeSO_4$ $7H_2O$ | 0.1 |
| $MgSO_4$ $7H_2O$ | 0.1 |
| $CaCO_3$ | 0.2 |
| starch | 2.5 |
| agar | 20. |

no pH adjustment, distilled $H_2O$ QS to 1.0 liter
Sterilization: 30 min, 121°C, 15-16 lb pressure

A4B Seed Medium

| | gm/liter |
|---|---|
| glucose | 15. |
| soybean meal | 15. |
| NaCl | 5. |
| $CaCO_3$ | 1. |

no pH adjustment, QS tap $H_2O$ to 1.0 liter

Vol/flask: 100 ml/500 ml Erlenmeyer flask closed with cotton plugs

Sterilization: 30 min, 121°C, 15-16 lb pressure

**Table 2**

**(Example 2) Thin-layer Chromatography of Extracted 72 Hr Fermentation Beer**

Thin-layer Chromatography Solvent Systems

| Fermentation Beer Solvent Extract | System 1* | | | System 2 ** | | | System 3*** | | |
|---|---|---|---|---|---|---|---|---|---|
| | *S. aureus* bioautography | Product Color | UV Absorbance | *S. aureus* bioautograhy | Product Color | UV Absorbance | *S. aureus* bioautography | Product Color | UV Absorbance |
| butanol extract (in methanol) | Rf .26 | yellow orange | Rf 0.26 | Rf .26 | yellow orange | Rf 0.26 | Rf .36 | yellow orange | Rf 0.36 |
| methylene chloride extract | | | | .08 | yellow orange | 0.08 | .16 | yellow orange | 0.16 |

Thin-layer Chromatography Solvent Systems for Analtech Silica Gel GF254 Plates

* ethyl acetate:formic acid:$H_2O$:isopropanol (14:2:1:1)
** methylene chloride:methanol:$H_2O$ (70:30:1)
*** butanol:acetic acid:$H_2O$ (3:1:1)

III. Isolation and Purification:

Benzanthrin A:

The whole broth (13 L) from Example 2 was adjusted to pH 6.5 and the mycelium was pelleted by centrifugation. The supernate was saturated with sodium chloride and extracted twice with 1/4 volume of n-butanol. The butanol was evaporated *in vacuo* leaving 14 g of an oily residue. This material was dissolved in a minimal amount of 50% aqueous methanol and charged onto a flash column packed with 90 g of C-8 capped silica gel (40 um) such as that made by J. T. Baker Chemical Co., Philipsburg, New Jersey, equilibrated in 0.01 M $NH_4HCO_3$ (pH 7.8). The activity was eluted with a step gradient consisting of: (1) $NH_4HCO_3$ buffer, (2) $NH_4HCO_3$ buffer - MeOH (20:80) and (3) MeOH. All active fractions were combined and further purified by prep HPLC (such as ODS-3 made by Whatman, Inc., Clifton, New Jersey) with 0.1% $Et_3N$ MeOH. Evaporation of the solvent afforded 45 mg of benzanthrin as a dark red amorphous solid giving a FAB (Fast Atom Bombardment) positive ion mass spectrum with $M+1^+$ 635 m/z, mass matched at 635.2957; theoretical for $C_{35}H_{43}N_2O_9$ is 635.2969.

Benzanthrins A or B

The whole broth (15 L) from Example 2 was pH adjusted to pH 6.5 and the mycelium was pelleted. The pH of the supernate was then adjusted to 8.5 and the supernate was extracted twice with 1/4 volumes of $CH_2Cl_2$. Evaporation of the solvent left 0.8 g of solid residue which was 18-fold more pure than the butanol extract above. Final purification and separation was achieved by countercurrent chromatography on an Ito planet coil centrifuge using

$MeOH-H_2O-CHCl_3-CCL_4$ (4:1:1:4 or 35:15:25:25). The fractions were assayed by HPLC:25 cm partisil propylaminocyano column (10 um particle size), 5 minute gradient ($EtCl_2$ to 1% $Et_3N$ MeOH) at 2 ml/min, $t_R$ (retention time) = 5.8 min for benzanthrin A and $t_R$ = 5.7 min for benzanthrin B. Those fractions containing pure materials were combined and the solvent was evaporated leaving 39 mg of benzanthrin A and 29 mg of benzanthrin B as dark red solids.

IV. Physical and Chemical Properties of Benzanthrins A and B:

Solubility:

At pH 8-9 the isolated compounds of the invention are soluble in lower alcohols, ethyl acetate, acetone, benzene, chloroform, methylene chloride, carbon tetrachloride, ethyl ether, tetrahydrofuran.

At pH 5-6 the isolated compounds of the invention are soluble in lower alcohols, dimethylsulfoxide and water.

Color Changes in Aqueous Alcohol:

At pH 5-6, red to orange.

At pH 8-9, blue to purple.

Ultraviolet-Visible (MeOH):

| | | λ max | | | |
|---|---|---|---|---|---|
| MeOH | A:230 | 260 | 304 | 428 | orange-red |
| | B:230 | 260 | 304 | 426 | |
| 0.1 N NaOH | A:230 | 245 | 290 | 510 | blue |
| | B:230 | 243 | 289 | 510 | |
| 0.1 N HCl | A:230 | 260 | 304 | 428 | orange-red |
| | B:230 | 260 | 304 | 426 | |

Degradations

Zinc dust distillation of benzanthrins A and B provided the same product which had UV and mass spectra

consistent with a benz[a]anthracene aromatic nucleus of structure A shown hereinbelow.

(A) 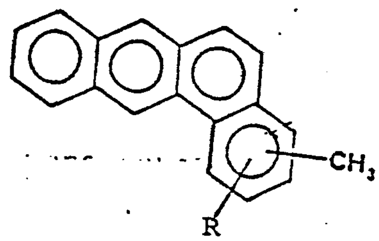

Ultraviolet (EtOH)

λ max

| | R | | | | | | | | | |
|---|---|---|---|---|---|---|---|---|---|---|
| A | $CH_3$ | 386 | 359 | 344 | 326 | 316 | 303 | 288 | 278 | 267 |
| (1.) | H | 385 | 359 | 342 | 328 | 314 | 300 | 287 | 273 | 268 |

A exhibited MS (EI): $M^{+\cdot}$256

(1.) 3-methyl-Benz[A]anthracene
Kuntsman, M. and L. Mitscher, J. Org. Chem., 31, 2920 (1966).

Diluted hydrochloric acid hydrolysis of benzanthrins A and B gave the same dark green solid C with an extended chromophore and mass spectrum as follows:

Ultraviolet - Visible (MeOH)

λ max

| | | | | | |
|---|---|---|---|---|---|
| B | 235 | 265 | 326 | 466 | 555 |

B exhibited MS (EI): $M^{+\cdot}$ 477, corresponding to $C_{27}H_{27}NO_7$.

(B) 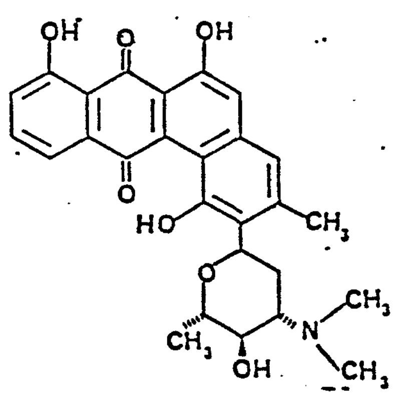

The infrared (IR) spectra of the compounds of the invention in $CDCl_3$ are shown in FIGURE 1. The observed absorption bands are consistent with the hypothesis that the compounds of the invention include a quinone structure.

The hydrogen nuclear magnetic resonance spectra of the compounds of the invention in $CD_2Cl_2$ are shown in FIGURE 2.

FIGURE 3 shows the carbon-13 nuclear magnetic resonance spectra of the compounds of the invention in $CD_2Cl_2$.

The NMR data are consistent with the hypothesis that the compounds of the invention include a quinone structure.

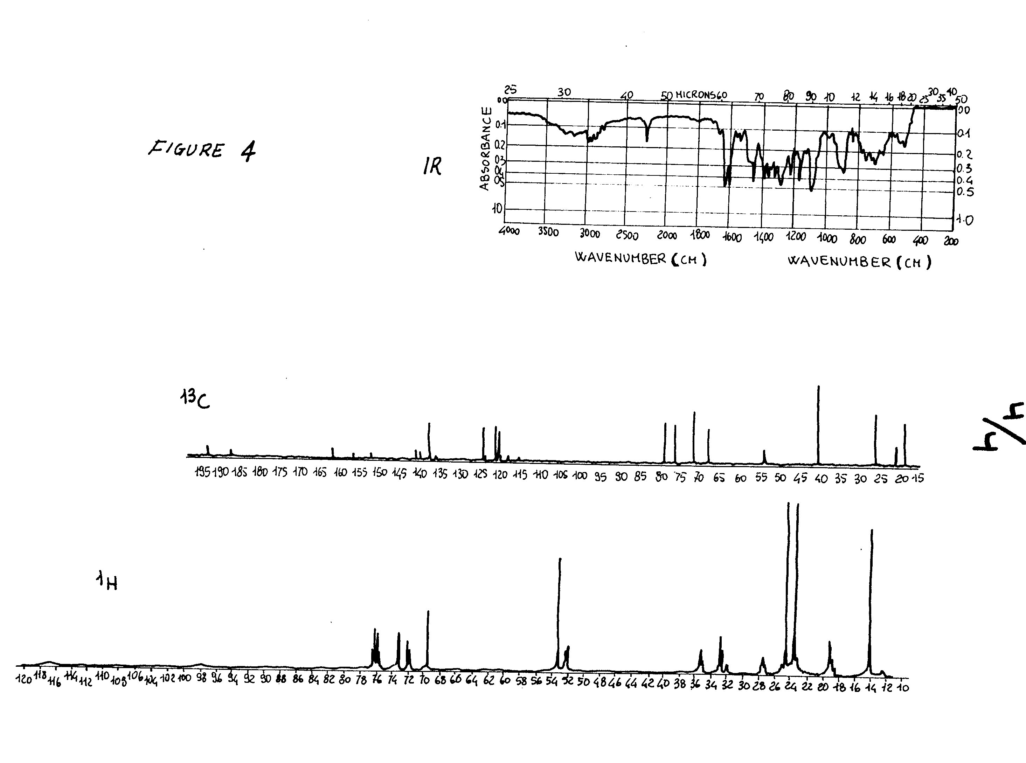

FIGURE 4 shows infrared and NMR data for the degradation compound, structure C hereinabove, which is a benzanthrin pseudoaglycone so described because it still possesses a sugar in the form of a C-glycoside linkage. This data reaffirms the hypothesis that the compounds of the invention include a quinone structure.

Acceptable Salts:

The present invention contemplates the pharmaceutically acceptable nontoxic acid addition salts of the compounds of the invention including the mineral acid addition salts such as hydrochloride, hydrobromide, hydroiodide, sulfate, sulfamate and phosphate and the organic addition salts such as maleate, acetate, citrate, oxalate, succinate, benzoate, tartrate, fumarate, malate, mandelate, ascorbate and the like.

## V. Synthetic Process for Making Benzanthrins

Protected benzanthrin pseudoaglycone compounds similar to structure B, are useful as intermediates for the synthetic conversion of either benzanthrin A to benzanthrin B or benzanthrin B to benzanthrin A.

The foregoing may be better understood from the following process schematic and examples, which are presented for purposes of illustration and are not intended to limit the scope of the inventive concepts.

(1)

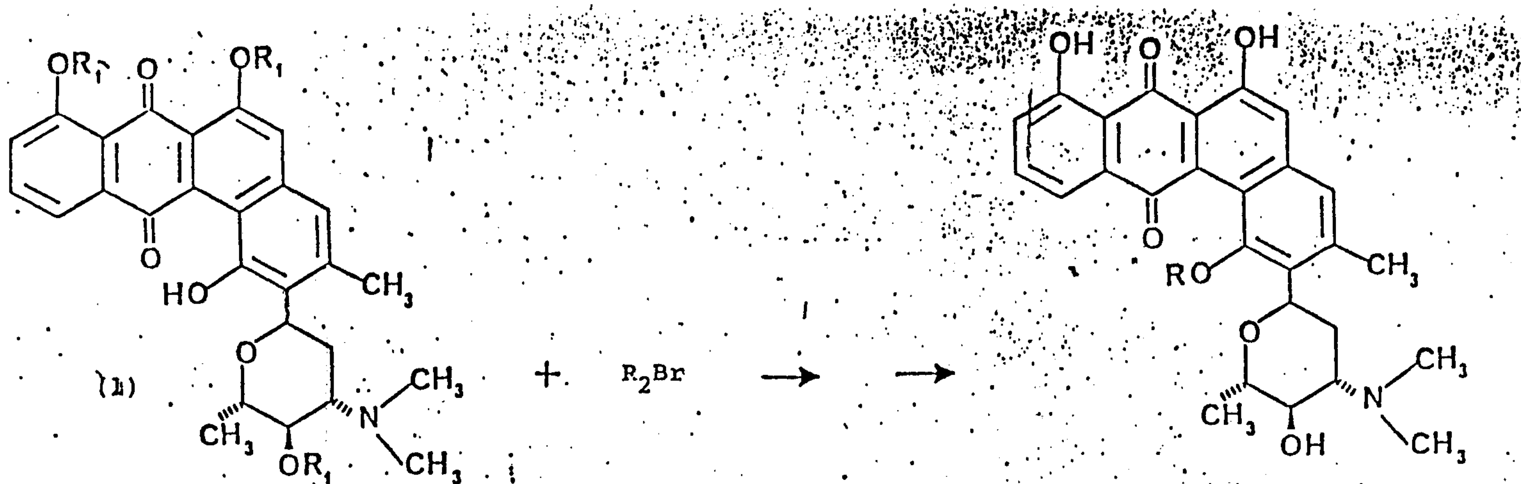


wherein $R_2$ is

wherein R is

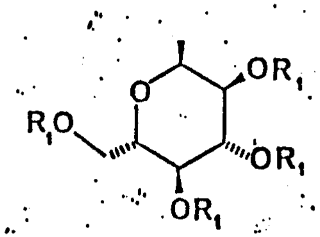

(3)

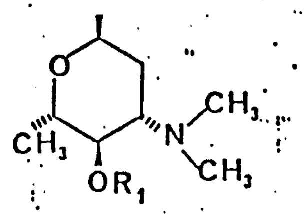

(4)

or

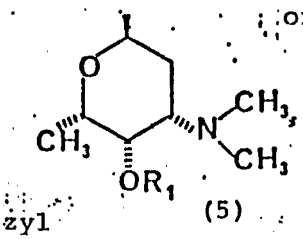

(5)

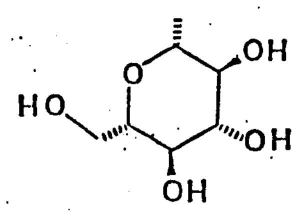

(6)

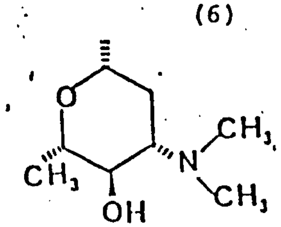

(7)

or

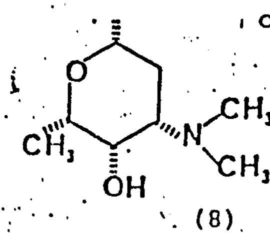

(8)

wherein $R_1$ is benzyl

and $R_1$ is benzyl

## Example 5

### 4',6,8-Tri-O-benzylbenzanthrin pseudoaglycone

A mixture of benzanthrin A (0.4 mmole), 4 mL of dimethylformamide (DMF), 0.7 mmole of potassium hydroxide and 0.25 g of finely ground drierite was stirred at room temperature for 30 minutes. This mixture was then cooled and 1.2 mmole of benzylbromide was added dropwise while keeping the reaction mixture below 70°C. The mixture was maintained at 65°C while stirring for an additional 1 1/2 hours, cooled to room temperature and filtered through celite. The solids were washed with DMF and the combined filtrates, concentrated *in vacuo* leaving an oily residue. The oily residue was taken up in 4 mL of anhydrous 1% methanolic hydrochloric acid and heated at 85°C for 15 minutes. The mixture was concentrated *in vacuo*, methanol was added and the mixture again concentrated *in vacuo* leaving the hydrochloride of the crude benzylated pseudoaglycone (1). Semipurified (1) was obtained by chromatography on amino reverse phase silica gel (100% water to 100% methanol gradient) affording free base of adequate purity for the reaction sequence below.

## Example 6

### 1-Glyco-benzanthrin pseudoaglycone

To a solution of the benzylated pseudoaglycone (1) (0.2 mmole) in 5 mL of dry methylene chloride, 0.5 g of freshly powdered drierite, 0.2 g of dried mercuric cyanide and 0.23 mmole of the sugar bromide (3) were added and the mixture heated under reflux for 20 hours with vigorous stirring. An additional 0.23 mmole of the sugar bromide (3) was added and the reaction mixture was stirred and heated for an additional 24 hours. The crude product mixture was filtered, the filtrate washed

with 5% sodium bicarbonate, dried, and concentrated to an oil. A major product (15 mg) was isolated by flash chromatography on silica gel (1% methanol-methylene chloride). The 15 mg above was dissolved in a solution made up of 0.36 mL of 0.2 M methanolic hydrochloric acid and 3.6 mL of methanol. This was treated with hydrogen (3 atm) over 5% palladium on carbon (11 mg) for 4 hours at room temperature. The mixture was filtered and the filtrated concentrated in vacuo to give crude (6) as the hydrochloride salt. The free base of (6) was obtained by passing the hydrochloride salt in methanol over amino reverse phase silica gel. The crude free base was then purified by counter current chromatography on a coil planet centrifuge in a biphasic system made from methanol-water-carbon tetrachloride-chloroform (4:1:4:1) to give a dark red solid: mass spec FAB $(M+1)^+$ matched as 607.2410, theoretical for $C_{33}H_{37}NO_{10}$ = 607.2417.

## Examples 7 and 8

Benzanthrins A and B may be interconverted by starting with the benzylated pseudoaglycone (1) derived from either A or B and carrying out the processes of Examples 5 and 6 with either sugar bromide (4) or (5) to give either (7) or (8), respectively, as a final product.

## VI. Antibacterial Properties:

The antibacterial activity of the compounds of the invention against a variety of microorganisms is illustrated in Table 3. Minimum inhibitory concentrations were determined by the agar dilution method using brain heart infusion agar with $10^{-5}$ CFU (colony forming units) as the inoculum.

TABLE 3

IN VITRO ACTIVITY OF BENZANTHRINS A and B

MIC (ug/ml)

| ORGANISMS | MIC (ug/ml) A | MIC (ug/ml) B |
|---|---|---|
| Staphylococcus aureus ATCC 6538P | 3.1 | 3.1 |
| Staphylococcus aureus CMX 686b | 3.1 | 1.56 |
| Staphylococcus aureus A5177 | 3.1 | 1.56 |
| Staphylococcus aureus 45 | 3.1 | 1.56 |
| Staphylococcus aureus 45 RAR2 | 3.1 | 3.1 |
| Staphylococcus epidermidis 3519 | 3.1 | 3.1 |
| Staphylococcus epidermidis 3519 RAR1 | 3.1 | 3.1 |
| Lactobacillus casei ATCC 7469 | 0.78 | 0.78 |
| Streptococcus faecium ATCC 8043 | 1.56 | 1.56 |
| Streptococcus bovis A5169 | 0.39 | 0.2 |
| Streptococcus agalactiae CMX 508 | 0.78 | 0.39 |
| Streptococcus pyogenes EES61 | 1.56 | 0.39 |
| Streptococcus pyogenes 930 | 0.2 | 0.2 |
| Sarcina lutea 9341 | 1.56 | -- |
| Escherichia coli Juhl | 100 | 100 |
| Escherichia coli SS | 3.1 | 6.2 |
| Escherichia coli DC-2 | 100 | 100 |
| Enterobacter aerogenes ATCC 13048 | 100 | 100 |
| Klebsiella pneumoniae ATCC 8045 | 100 | 100 |
| Pseudomonas aeruginosa 5007 | 100 | 100 |
| Acinetobacter CMX 669 | 100 | -- |
| Pseudomonas cepacia 2961 | 100 | 100 |

* Determined by the agar dilution procedure using Brain Heart Infusion agar with $10^5$ CFU as the inoculum.

It is evident from the above table that the benzanthrins have broad activity against Gram-positive bacteria.

In view of their excellent antibacterial activity, the compounds of the invention can be used alone or in combination with other antibacterial agents to prevent the growth of, or reduce the number of, organisms present in various environments. For example, the compounds of the invention can be used in papermill systems to inhibit the growth of cultures which are known to produce slime in such systems. The compounds of the invention are also useful as oil preservatives, for example, as a bacteriostatic agent for inhibiting the growth of Staphylococcus aureus which is known to cause spoilage in cutting oils. Also, the compounds of the invention are useful in wash solutions for sanitation purposes, as in the washing of hands and the cleaning of equipment, floors, or furnishings of contaminated rooms or laboratories; the compounds of the invention are also useful as an industrial preservative, for example, as a bacteriostatic rinse for laundered clothes and for impregnating paper and fabrics, and the compounds of the invention are useful for suppressing the growth of sensitive organisms in plate assays and other microbiological media. The compounds of the invention can be used in treating infections caused by susceptible organisms in warm-blooded animals.

## VII. Cytotoxic Properties

The cytotoxic activity of the compounds of the invention can be tested by a KB antitumor test. KB cells are derived from human epidermoid carcinoma of the mouth and are cultivated on Eagel's basal medium plus 10% serum. Stock cells are fed 24 hours before testing. Test drug is added on day 0 or day 1. Results

are expressed as the dose which inhibits growth to 50% of control growth by 3 days after drug addition.

When the KB antitumor test was conducted on benzanthrins A and B, the following test data resulted:

| | $ED_{50}$ (mg/ml) | |
|---|---|---|
| Tumor Cell | Benzanthrin A | Benzanthrin B |
| 9KB | 0.1 - 0.3 | 0.2 - 0.3 |

Generally compounds are considered active cytotoxic agents if their $ED_{50}$ is less than 4 mg/ml. Accordingly, it is apparent that the compounds of the invention are extremely active.

The foregoing is merely illustrative of the invention and is not intended to limit the invention to the disclosed compounds. Variations and change which are obvious to one skilled in the art are intended to be within the scope and nature of the invention which are defined in the appended claims.

CLAIMS

1. A process for producing a benzanthrin compound which comprises culturing a microorganism belonging to the species Nocardia lurida having the ability to produce a benzanthrin compound in a nutrient medium and accumulating a benzanthrin compound in said medium.

2. A process according to Claim 1 wherein said microorganism is Nocardia lurida B7 NRRL 15820 and wherein said microorganism is cultured at a temperature of 23-28°C and a pH of 6-9.

3. The benzanthrin A of the process of Claim 1.

4. The benzanthrin B of the process of Claim 1.

5. A compound of the formula:

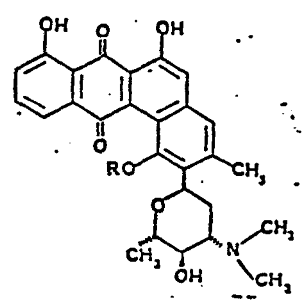

wherein R is

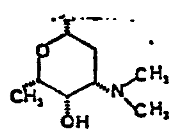

or

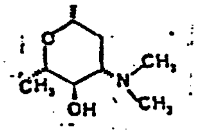

6. A composition having antibacterial activity which comprises a dark red solid characterized by:

(a) molecular weight of 634;

(b) molecular formula of $C_{35}H_{42}N_2O_9$;

(c) an infrared absorption spectrum as shown in accompanying FIGURE 1a,

a hydrogen nuclear magnetic resonance spectrum as shown in accompanying FIGURE 2a, and

carbon-13 nuclear magnetic resonance spectrum as shown in accompanying FIGURE 3a; or

an infrared absorption spectrum as shown in accompanying FIGURE 1b,

a hydrogen nuclear magnetic resonance spectrum as shown in accompanying FIGURE 2b, and

carbon-13 nuclear magnetic resonance spectrum as shown in accompanying FIGURE 3b;

or a pharmaceutically acceptable salt thereof.

7. A composition having cytotoxic activity which comprises a dark red solid characterized by:

(a) molecular weight of 634;

(b) molecular formula of $C_{35}H_{42}N_2O_9$;

(c) an infrared absorption spectrum as shown in accompanying FIGURE 1a,

a hydrogen nuclear magnetic resonance spectrum as shown in accompanying FIGURE 2a, and

carbon-13 nuclear magnetic resonance spectrum as shown in accompanying FIGURE 3a; or

an infrared absorption spectrum as shown in accompanying FIGURE 1b,

a hydrogen nuclear magnetic resonance spectrum as shown in accompanying FIGURE 2b, and

carbon-13 nuclear magnetic resonance spectrum as shown in accompanying FIGURE 3b;

or a pharmaceutically acceptable salt thereof.

8. A substantially biologically pure culture of the microorganism <u>Nocardia</u> <u>lurida</u> B7 NRRL 15820 and mutations thereof, said culture being capable of producing a benzanthrin compound upon culturing of said microorganism in a nutrient medium.

9. A compound of the formula:

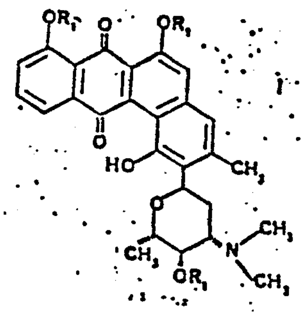

wherein $R_1$ is benzyl.

10. A process of making the compounds of Claim 7 by reacting the compound of the formula:

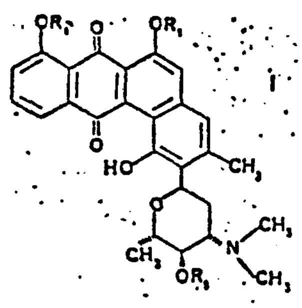

with a sugar bromide of the formula: $R_2Br$

Wherein $R_2$ is

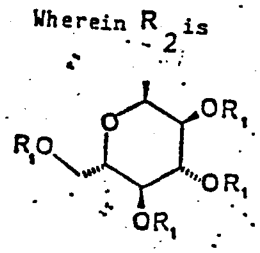

or

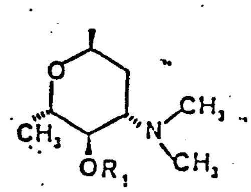

or

and $R_1$ is benzyl.

0180045

1/4

FIGURE 1 : IR

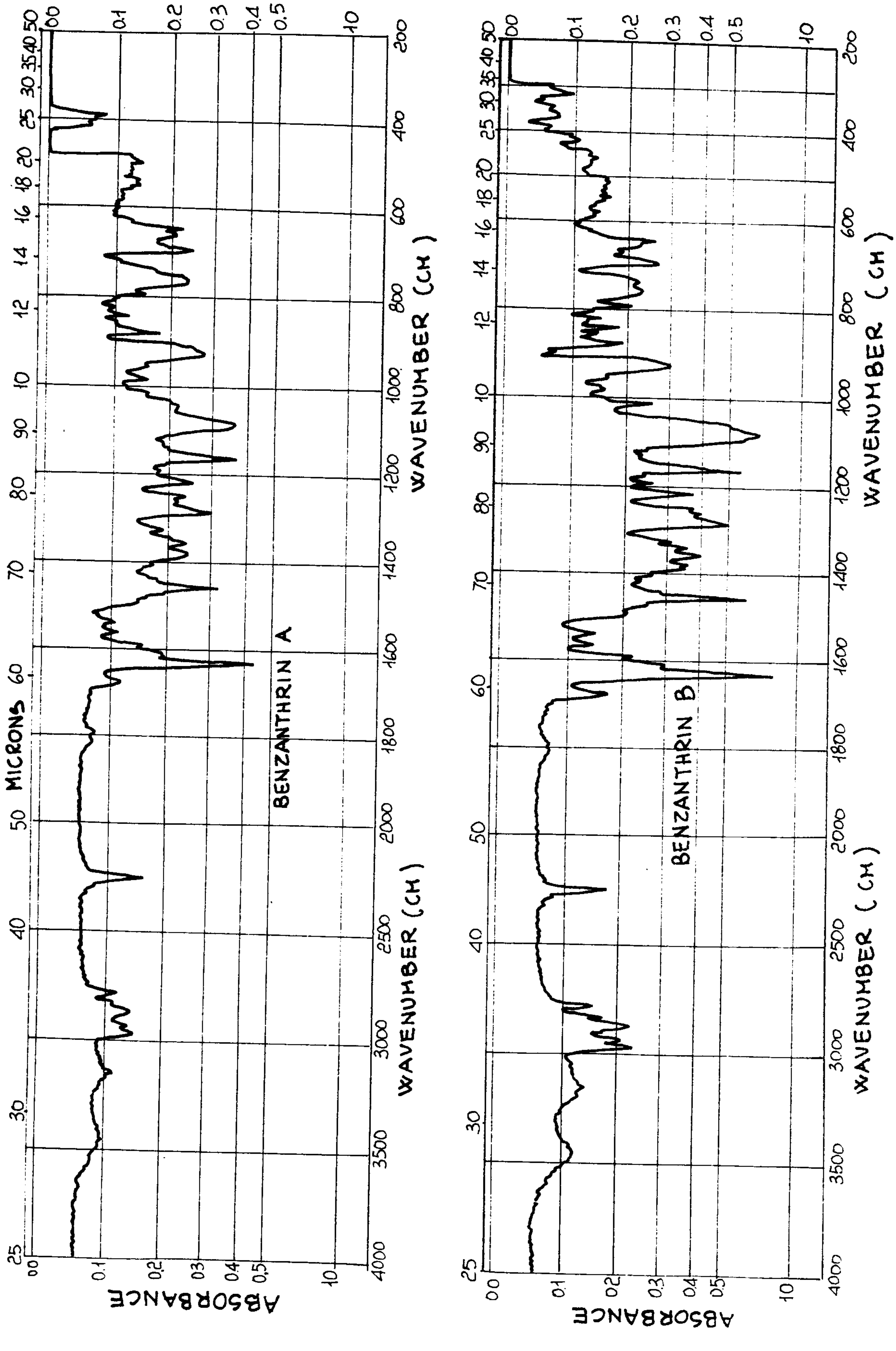

0180045

2/4

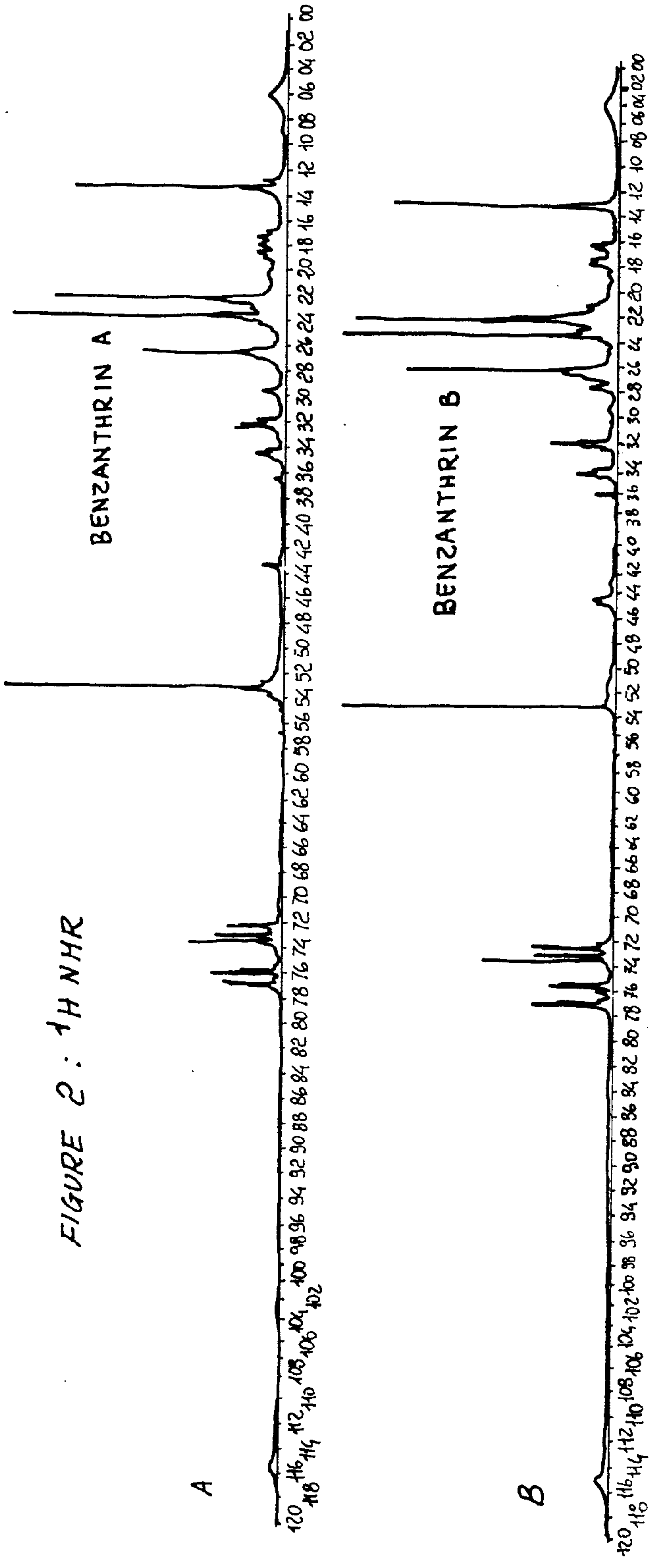


FIGURE 2 : $^1H$ NMR

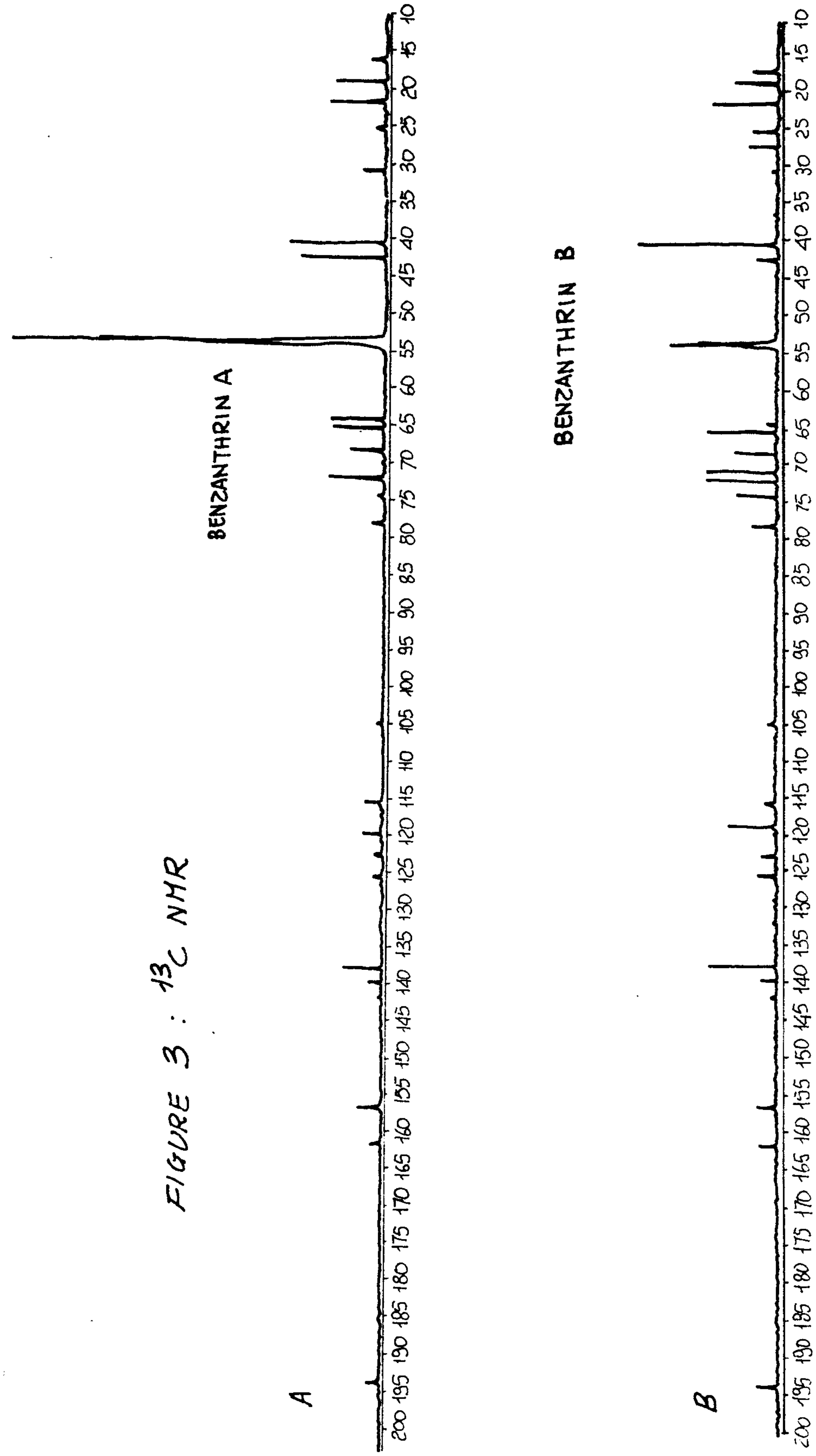


FIGURE 3 : $^{13}C$ NMR

0180045

4/4

FIGURE 4

IR

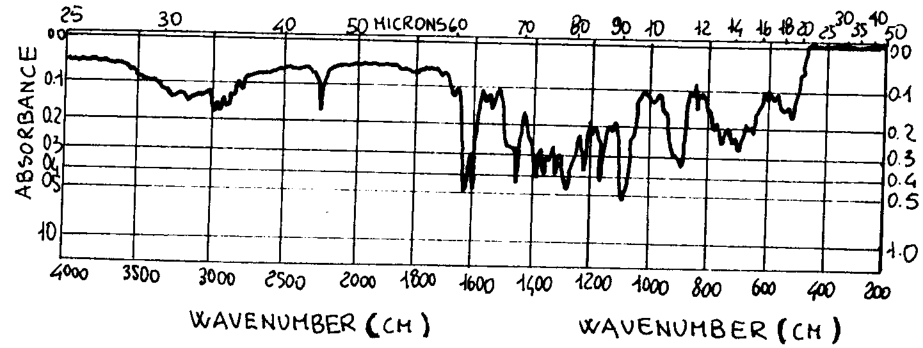


$^{13}C$

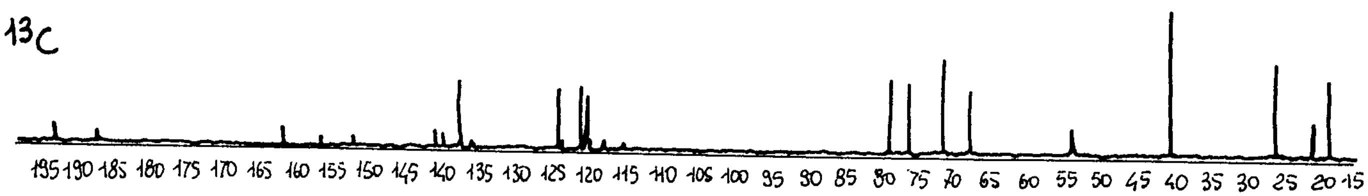

$^1H$